# EUROPÄISCHE PATENTSCHRIFT

(11) **EP 1 210 958 B1**
(45) Veröffentlichungstag und Bekanntmachung des Hinweises auf die Patenterteilung: **09.03.2005**
(21) Anmeldenummer: 00126321.9
(22) Anmeldetag: 01.12.2000
(51) Int. Cl.: A61M 25/00, A61L 29/04, A61L 29/06, A61L 29/14

(54) **Ballon für medizinische Geräte und Verfahren zur Herstellung von Ballonen für medizinische Geräte**
Balloon for medical devices and method for manufacturing balloons for medical devices
Ballon pour dispositifs médicaux et procédé pour la fabrication de ballons pour dispositifs médicaux

(43) Veröffentlichungstag der Anmeldung: 05.06.2002
(73) Patentinhaber: Bavaria Medizin Technologie GmbH, D-82234 Wessling/Oberpfaffenhofen (DE)
(72) Erfinder: Sauerteig, Knut, 86911 Diessen (DE)
(74) Vertreter: Hofstetter, Alfons J., Dr.rer.nat.

(56) Entgegenhaltungen:
- EP-A- 0 436 501
- WO-A-99/53986
- US-A- 5 500 181
- US-A- 5 533 968

## Beschreibung

Die vorliegende Erfindung betrifft einen Ballon für medizinische Geräte, insbesondere für Katheter, bestehend aus einem biokompatiblen und biostabilen Material, wobei sich der Ballon unter Druck ausdehnt und einen vorbestimmten Nominaldurchmesser bei einem definierten Nenndruck aufweist. Des weiteren betrifft die vorliegende Erfindung ein Verfahren zur Herstellung von Ballonen für medizinische Geräte und einen medizinischen Katheter mit einem schlauch- oder rohrförmigen Katheterkörper und mindestens einem an dem Katheterkörper angeordneten Ballon, wobei der Ballon flüssigkeits- und gasleitend mit dem Katheterkörper verbunden ist und der Katheterkörper und der Ballon aus einem biokompatiblen und biostabilen Material bestehen, wobei sich der Ballon unter Druck ausdehnt und einen vorbestimmten Nominaldurchmesser bei einem definierten Nenndruck aufweist.

Ballone für medizinische Geräte sind bekannt. Ein Ballon gemäß dem Oberbegriff des Anspruchs 1 und ein Verfahren gemäß dem Oberbegriff des Anspruchs 5 sind aus WO-A- 99/53986 bekannt. Ferner beschreibt das europäische Patent EP 0 436 501 B1 einen Ballon für eine medizinische Vorrichtung, wobei dieser Ballon eine Schlauchlänge umfasst, welche mechanisch in Radial- und Longitudinalrichtung gedehnt worden ist und aus einem Nylon- oder Polyamidmaterial besteht. Der bekannte Ballon weist ein nicht-ausgeweitetes Arbeitsprofil mit einer vorbestimmten Größe auf, zu welcher sich der Ballon ohne dessen signifikante Ausdehnung aufbläst. Des weiteren weist der Ballon ein Expansionsprofil mit einer maximal aufgeblasenen Größe auf, zu welcher sich der Ballon ausdehnt, ohne während der Verwendung zu zerbersten, wobei die maximal aufgeblasene Größe größer ist als die vorbestimmte Größe des nicht-ausgeweiteten Arbeitsprofils.

Die Größe des nicht-ausgeweiteten Arbeitsprofils entspricht üblicherweise dem Nominaldurchmesser des Ballons für medizinische Geräte. Dieser Nominaldurchmesser wird bei einem vordefinierten Nenndruck erreicht. Die maximal aufgeblasene Größe erreicht der Ballon bei einem maximal garantierten Druck bzw. Enddruck. Der Druckbereich zwischen dem Nenndruck und dem Enddruck wird üblicherweise als Arbeitsbereich des Ballons bezeichnet.

Des weiteren werden Ballone für medizinische Geräte nach ihrer Druck-Durchmesser-Charakteristik unterschieden. Die Unterteilung erfolgt in sogenannte compliant, semi-compliant und non-compliant Ballone. Damit wird die kontrollierte Überdehnbarkeit der Ballone in dem genannten Arbeitsbereich beschrieben. Üblicherweise wird so mit dem Begriff semi-compliant ein Ballon definiert, der im Arbeitsbereich zwischen Nenndruck und Enddruck seinen Durchmesser um eine halbe Größe erweitert. Dies bedeutet, daß z. B. ein Ballon mit 3,0 mm Durchmesser sich bis auf ca. 3,25 mm überdehnen lässt, da die nächste Ballongröße 3,5 mm ist. Bei einem compliant Ballon beträgt die Überdehnbarkeit eine ganze Ballongröße, nämlich z. B. von 3,0 mm Durchmesser bei Nenndruck auf 3,5 mm Durchmesser bei Enddruck.

Bei bekannten semi-compliant Ballonen liegen die Nenndrücke bei 6 oder 8 bar und die Enddrücke bzw. maximal empfohlene Arbeitsdrücke bei 12 bis 18 bar. Bei verschiedenen medizinischen Anwendungen wird aber das Erreichen des Nominaldurchmessers bei deutlich höheren Drücken gefordert. Werden nunmehr die bekannten Ballone, deren Nominaldurchmesser bei 6 bis 8 bar erreicht werden, eingesetzt, vergrößern diese bei höheren Anwendungsdrücken ihren Durchmesser. Dies wiederum bedeutet, dass der Anwender den für eine Applikation (z. B. Implantieren eines Stents) optimalen Arbeitsdruck nur mit einem bereits über den Nenndurchmesser überdehnten Ballon erzielen kann, was bei medizinischen Anwendungen zu Komplikationen führen kann.

Es ist daher Aufgabe der vorliegenden Erfindung, einen Ballon für medizinische Geräte der eingangs genannten Art bereitzustellen, der die genannten Nachteile des Standes der Technik überwindet und dennoch semi-compliant Eigenschaften aufweist.

Es ist weiterhin Aufgabe der vorliegenden Erfindung, ein Verfahren zur Herstellung von Ballonen für medizinische Geräte bereitzustellen, das die physikalischen Charakteristika des erfindungsgemäßen Ballons gewährleistet.

Gelöst werden diese Aufgaben durch einen Ballon und ein Verfahren gemäß den Merkmalen der unabhängigen Ansprüche 1 und 7.

Vorteilhafte Ausgestaltungen der Erfindung sind in den Unteransprüchen beschrieben.

Ein erfindungsgemäßer Ballon für medizinische Zwecke ist vorteilhafterweise derart ausgebildet, dass der Nominaldurchmesser bei einem höheren Druck als den bekannten Ballonen liegt und mehr an dem Anwendungsdruck orientiert ist. Der Durchmesser des Ballons vergrößert sich dabei im Druckbereich zwischen Nenndruck und maximal empfohlenem Arbeitsdruck um weniger oder gleich 5 %, wobei der Nominaldurchmesser des Ballons zwischen 1 und 6 mm beträgt und der Nenndruck zwischen 10 und 14 bar liegt. Dadurch ist einerseits gewährleistet, daß der Ballon bei höheren Nenndrücken als bisher bekannt den Nominaldurchmesser erreicht, insbesondere bei Drücken, wie sie für verschiedene Anwendungen gefordert werden. Andererseits ist eine Überdehnung gewährleistet, die den Ballon für einen größeren Durchmesser und somit auch Anwendungsbereich einsatzfähig macht.

In der nachfolgenden Tabelle sind die Charakteristika und Merkmale des erfindungsgemäßen Ballons, insbesondere die Erreichung des Nominaldurchmessers bei einem Druck von 12 bar beispielhaft dargestellt. Der maximal empfohlene Arbeitsdruck liegt für dieses Beispiel bei 18 bar für Ballondurchmesser von 2,0 bis 4,0 mm.

**Tabelle**

| Druck (bar) Durchmesser (mm) | 12 | 14 | 16 | 18 |
|---|---|---|---|---|
| 2,00 | 2,00 | 2,03 | 2,06 | 2,10 |
| 2,50 | 2,50 | 2,54 | 2,58 | 2,62 |
| 3,00 | 3,00 | 3,05 | 3,10 | 3,15 |
| 3,50 | 3,50 | 3,55 | 3,61 | 3,67 |
| 4,00 | 4,00 | 4,06 | 4,13 | 4,20 |

Üblicherweise besteht der erfindungsgemäße Ballon aus einem Polyamidmaterial, Polyurethanmaterial oder dessen Copolymeren, Kautschuk, Silikon, Polyolefin oder deren Copolymeren.

Die Erfindung betrifft weiterhin einen medizinischen Katheter mit einem schlauchförmigen Katheterkörper und mindestens einem an dem Katheterkörper angeordneten Ballon, wobei der Ballon flüssigkeits- und gasleitend mit dem Katheterkörper verbunden ist und der Katheterkörper und der Ballon aus einem biokompatiblen und biostabilen Material bestehen, wobei sich der Ballon unter Druck ausdehnt und einen vorbestimmten Nominaldurchmesser bei einem definierten Nenndruck aufweist. Erfindungsgemäß ist der Ballon derart ausgebildet, dass sich der Durchmesser des Ballons dabei im Druckbereich zwischen Nenndruck und maximal empfohlenem Arbeitsdruck um weniger oder gleich 5 % vergrößert, wobei der Nominaldurchmesser des Ballons zwischen 1 und 6 mm beträgt und der Nenndruck zwischen 10 und 14 bar liegt. Der maximal empfohlene Arbeitsdruck liegt dabei üblicherweise zwischen 16 und 18 bar. Auf Grund dieser Durchmesser- und Druckcharakteristik ist es möglich, nahezu alle medizinischen Anwendungsbereiche für derartige Katheter abzudecken. Der medizinische Katheter besteht dabei insbesondere aus einem Polyamidmaterial, Polyurethanmaterial oder dessen Copolymeren, Kautschuk, Silikon, Polyolefin oder deren Copolymeren. Der Ballon ist üblicherweise mit dem schlauch- oder rohrförmigen Katheterkörper einstückig ausgebildet

Eine erfindungsgemäße Verwendung des beschriebenen medizinischen Katheters erfolgt in einem Positionier- und Implantiersystem für Stents. Bei derartigen Systemen ist es für den Anwender sehr wichtig zu wissen, welchen Durchmesser der Ballon bei entsprechend hohen Implantationsdrücken aufweist.

Ein erfindungsgemäßes Verfahren zur Herstellung von Ballonen für medizinische Geräte umfasst folgende Schritte:
(a) Radiales Dehnen eines Schlauchelements aus einem biokompatiblen und biostabilen Kunststoff mit einem vorbestimmten Anfangsdurchmesser; anschließend
(b) axiales Strecken des radial gedehnten Schlauchelements entlang der Schlauchachse, wobei der biaxiale Dehn- und Streckvorgang gemäß den Verfahrensschritten (a) und (b) bei Temperaturen die 25° - 45 °C unterhalb der Kristallitschmelztemperatur des verwendeten Kunststoffmaterials liegen, und mit einem Formdruck zwischen 25 und 40 bar durchgeführt wird. Dadurch ist gewährleistet, dass der resultierende Ballon sich in dem Arbeitsbereich zwischen Nenndruck und dem maximal empfohlenem Arbeitsdruck um nur weniger oder gleich 5% des Nominaldurchmessers vergrößert, wobei der Nominaldurchmesser des Ballons zwischen 1,0 und 6,0 mm beträgt und der Nenndruck zwischen 10 und 14 bar liegt.

In einer weiteren vorteilhaften Ausgestaltung des erfindungsgemäßen Verfahrens wird das Verfahren bei Temperaturen die 30° - 35 °C unterhalb der Kristallitschmelztemperatur des verwendeten Kunststoffmaterials liegen, und mit einem Formdruck zwischen 30 und 35 bar durchgeführt. Zudem ist das Verfahren üblicherweise ein Blasformverfahren. Es ist aber auch denkbar, dass andere geeignete Verfahren zur Anwendung kommen.

## Patentansprüche

1. Ballon für medizinische Geräte, insbesondere für Katheter, bestehend aus einem biokompatiblen und biostabilen Material, wobei sich der Ballon unter Druck ausdehnt und einen vorbestimmten Nominaldurchmesser bei einem definierten Nenndruck aufweist,
**dadurch gekennzeichnet,**
**dass** der Ballon derart ausgebildet ist, dass sich der Nominaldurchmesser des Ballons in einem Druckbereich der höher ist als der Nenndruck und niedriger ist als ein maximal empfohlener Arbeitsdruck, um weniger oder gleich 5% des Nominaldurchmessers vergrößert, wobei der Nominaldurchmesser des Ballons zwischen 1,0 und 6,0 mm beträgt und der Nenndruck zwischen 10 und 14 bar liegt.

2. Ballon nach Anspruch 1,
**dadurch gekennzeichnet,**
**dass** der maximal empfohlene Arbeitsdruck zwischen 16 und 18 bar liegt.

3. Ballon nach Anspruch 1 oder 2,
**dadurch gekennzeichnet,**
**dass** der Ballon aus einem Polyamidmaterial oder dessen Copolymeren, Polyurethanmaterial, Kautschuk, Silikon, Polyolefinen oder deren Copolymeren besteht.

4. Medizinischer Katheter mit einem schlauch- oder rohrförmigen Katheterkörper und mindestens einem an dem Katheterkörper angeordneten Ballon gemäß mindestens einem der Ansprüche 1 bis 3, wobei der Ballon flüssigkeits- und gasleitend mit dem Katheterkörper verbunden ist und der Katheterkörper und der Ballon aus einem biokompatiblen und biostabilen Material bestehen, wobei sich der Ballon unter Druck ausdehnt und einen vorbestimmten Nominaldurchmesser bei einem definierten Nenndruck aufweist.

5. Verfahren zur Herstellung von Ballonen für medizinische Geräte gemäß den Ansprüchen 1 bis 3, wobei das Verfahren folgende Schritte umfasst:
a) Radiales Dehnen eines Schlauchelements aus einem biokompatiblen und biostabilen Kunststoff mit einem vorbestimmten Anfangsdurchmesser;
b) axiales Strecken des radial gedehnten Schlauchelements entlang der Schlauchachse,
**dadurch gekennzeichnet,**
**dass** der biaxiale Dehn- und Streckvorgang gemäß den Verfahrensschritten a) und b) bei Temperaturen die 25° - 45 °C unterhalb der Kristallitschmelztemperatur des verwendeten Kunststoffmaterials liegen, und mit einem Formdruck zwischen 25 und 40 bar durchgeführt wird.

6. Verfahren nach Anspruch 5,
**dadurch gekennzeichnet,**
**dass** das Verfahren bei Temperaturen die 30° - 35 °C unterhalb der Kristallitschmelztemperatur des verwendeten Kunststoffmaterials liegen, und mit einem Formdruck zwischen 30 und 35 bar durchgeführt wird.

7. Verfahren nach Anspruch 5 oder 6,
**dadurch gekennzeichnet,**
**dass** das Verfahren ein Blasformverfahren ist.

8. Positionier- und Implantiersystem für Stents mit einem medizinischen Katheter gemäß Anspruch 4.

## Claims

1. Balloon for medical devices, especially for catheters, consisting of a biocompatible and biostable material, the balloon expanding under pressure and having a predetermined nominal diameter under defined nominal pressure,
**characterized in that**
the balloon is formed such that the nominal diameter of the balloon increases by less or equal to 5% of the nominal diameter in a pressure range higher than the nominal pressure and lower than a maximum recommended operating pressure, wherein the nominal diameter of the balloon is between 1.0 and 6.0 mm and the nominal pressure is between 10 and 14 bar.

2. Balloon according to claim 1,
**characterized in that**
the maximum recommended operating pressure is between 16 and 18 bar.

3. Balloon according to claim 1 or 2,
**characterized in that**
the balloon is made of a polyamide material or copolymers thereof, polyurethane material, rubber, silicone, polyolefins or copolymers thereof.

4. Medical catheter having a hose- or tube-shaped catheter body and at least one balloon disposed on the catheter body according to at least one of claims 1 to 3, wherein the balloon is connected to the catheter body in liquid and gas conducting manner, and the catheter body and the balloon are made of biocompatible and biostable material, wherein the balloon expands under pressure and has a predetermined nominal diameter under a defined nominal pressure.

5. Method for producing balloons for medical devices according to claims 1 to 3, the method including the following steps:
a) radially expanding a hose member of biocompatible and biostable plastics having a predetermined initial diameter;
b) axially stretching the radially expanded hose member along the hose axis,
**characterized in that**
the biaxial expanding and stretching procedure is performed according to method steps a) and b) at temperatures 25° - 45°C below the crystallite melting temperature of the used plastic material and with a mold pressure between 25 and 40 bar.

6. Method according to claim 5,
**characterized in that**
the method is performed at temperatures 30° - 35°C below the crystallite melting temperature of the used plastic material and with a mold pressure between 30 and 35 bar.

7. Method according to claim 5 or 6,
**characterized in that**
the method is a blow molding technique.

8. Positioning and implanting system for stents with a medical catheter according to claim 4.

## Revendications

1. Ballon pour appareils médicaux, notamment des sondes, réalisé en un matériau biocompatible et biostable, le ballon se dilatant sous l'effet de pression et présentant un diamètre nominal prédéterminé avec une pression nominale définie,
**caractérisé en ce que**
le ballon est conçu de manière que dans une plage de pression supérieure à la pression nominale et inférieure à une pression de service maxi. recommandée le diamètre nominal du ballon augmente d'une valeur inférieure ou égale à 5 % du diamètre nominal, le diamètre nominal étant compris entre 1,0 et 6,0 mm, la pression nominale étant comprise entre 10 et 14 bars.

2. Ballon selon la revendication 1,
**caractérisé en ce que**
la pression maxi. recommandée est comprise entre 16 et 18 bars.

3. Ballon selon la revendication 1 ou 2,
**caractérisé en ce que**
le ballon est fait en un polyamide ou ses copolymères, un polyurethane, du caoutchouc, silicone, des polyoléfines ou leurs copolymères.

4. Sonde médicale comprenant un corps de sonde conçu sous forme de tuyau ou de tube et au moins un ballon disposé sur le corps de la sonde selon au moins l'une des revendications 1 à 3, le ballon étant relié en connexion conductrice de liquides et de gaz au corps de la sonde, le corps de la sonde et le ballon étant réalisés en un matériau biocompatible et biostable, le ballon se dilatant sous l'effet de compression et présentant un diamètre nominal prédéfini avec une pression nominale définie.

5. Procédé de fabrication de ballons pour dispositifs médicaux selon les revendications 1 à 3, le procédé comprenant les étapes suivantes:
a) Dilatation radiale d'un élément tubulaire réalisé en une matière plastique biocompatible et biostable et ayant un diamètre initial prédéfini;
b) étirage axial de l'élément tubulaire dilaté axialement selon l'axe du tube,
**caractérisé en ce que**
l'opération de dilatation et d'étirage biaxiaux selon les étapes du procédé a) et b) est effectuée à des températures inférieures de 25 à 45°C à la température de fusion des cristallites du matériau plastique mis en oeuvre et avec une pression de formage comprise entre 25 et 40 bars.

6. Procédé selon la revendication 5,
**caractérisé en ce que**
que le procédé est effectué à des températures inférieures de 30 à 35°C à la température de fusion des cristallites du matériau plastique mis en oeuvre et avec une pression de formage comprise entre 30 et 35 bars.

7. Procédé selon la revendication 5 ou 6,
**caractérisé en ce que**
le procédé est un procédé à soufflage.

8. Système de positionnement et d'implantation d'endoprothèses à l'aide d'une sonde médicale selon la revendication 4.
